# EUROPEAN PATENT APPLICATION

(11) **EP 4 144 373 A1**
(43) Date of publication of application: **08.03.2023**
(21) Application number: 21795269.6
(22) Date of filing: 14.04.2021
(51) Int. Cl.: A61K 45/06, A61K 31/403, A61K 31/381, A61K 31/7048, A61K 31/382, A61K 31/35, A61K 31/401, A61P 3/10, A61P 9/00, A61P 13/12, A61P 1/16

(54) **DRUG COMBINATION FOR TREATING DIABETES MELLITUS AND COMPLICATIONS THEREOF AND PHARMACEUTICAL COMPOSITION OF DRUG COMBINATION**

(30) Priority: 30.04.2020 CN 202010362374
(71) Applicant: Chengdu Chipscreen Pharmaceutical Co., Ltd, Chengdu, Sichuan 610041 (CN)
(72) Inventor: HUANG, Shengjian, Chengdu, Sichuan 610041 (CN); LU, Xianping, Chengdu, Sichuan 610041 (CN); PAN, Desi, Chengdu, Sichuan 610041 (CN); LIAO, Guoqiang, Chengdu, Sichuan 610041 (CN); ZHAO, Yiru, Chengdu, Sichuan 610041 (CN)
(74) Representative: Colombo, Stefano Paolo
(86) International application number: PCT/CN2021/087266
(87) International publication number: WO 2021/218638

(57) **Abstract**

Use of a component (i) represented by a general formula (I) or a pharmaceutically acceptable salt or isomer of the compound, in combination with a component (ii) of a SGLT2 inhibitor in the manufacture of a medicament for the prevention and/or treatment of diabetes mellitus and/or a diabetic complication, as well as a pharmaceutical composition comprising the component (i) and the component (ii).

## Description

### Technical Field

The present disclosure relates to the field of biotechnology and specifically relates to a drug combination and pharmaceutical composition thereof for the treatment of diabetes mellitus and complications thereof.

### Background Art

Diabetes mellitus is a disease caused by multiple genetic disorders, affecting a significant portion of the global population currently. It is mainly divided into two types: (1) type I diabetes or insulin-dependent diabetes mellitus (IDDM), in which patients secrete little or no insulin, and (2) type II diabetes or non-insulin-dependent diabetes mellitus (NIDDM). The core problem of type II diabetic patients is insulin resistance, which means that peripheral tissues such as muscles, liver and adipose tissues become impaired in the insulin-stimulated blood glucose absorption and metabolism, which in turn increases the burden of insulin secretion by the pancreas and eventually leads to progressive decline or even loss of islet function. 90% of diabetic patients have type II diabetes. This abnormal metabolic disease is characterized by hyperglycemia and causes a variety of complications such as cardiovascular disease, nephropathy, retinopathy, liver disease, and neurological disease.

The therapy for type II diabetes mellitus usually includes lifestyle management and medication. There are at least 8 major classes of oral or injectable therapeutic agents other than insulin according to the therapeutic mechanisms, but there are limitations in the effective population and long-term efficacy of monotherapy with existing drugs.

In addition, patients with diabetes have a higher risk of cardiovascular, renal, and hepatic complications than other populations, while the available therapeutic agents are all able to control blood glucose but are different in the effectiveness of preventing and controlling these complications.

The development of a drug combination that can effectively prevent and/or treat diabetes mellitus and its complications is of great importance.

### Contents of the Disclosure

The purpose of the present disclosure is to provide a drug combination and a pharmaceutical composition thereof with excellent effectiveness in preventing and/or treating diabetes mellitus and its complications.

To solve the above technical problem, in a first aspect, the present disclosure provides use of a component (i) in combination with a component (ii) in the manufacture of a medicament for the prevention and/or treatment of diabetes mellitus and/or a diabetic complication, wherein the diabetes mellitus is preferably type II diabetes mellitus, and the diabetic complication includes cardiovascular disease, renal disease and liver disease:
the component (i) is a compound having the formula shown in general Formula (I), or a pharmaceutically acceptable salt or isomer thereof; the component (ii) is a sodium-dependent glucose transporter 2 (SGLT2) inhibitor: 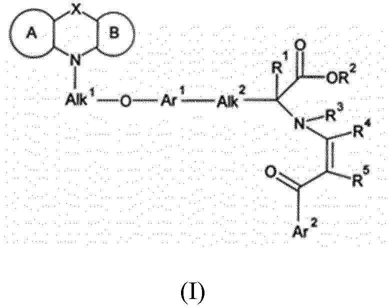
wherein ring A and ring B are respectively benzene rings, with no substituent or one or more substituents on the ring, and the substituent is fluorine, alkyl or alkoxy;
X is a covalent bond, O or S;
R¹ is H or alkyl;
R² is H or alkyl;
R³ is H or alkyl;
R⁴ and R⁵ are H or alkyl, respectively, or R⁴ and R⁵ together form a benzene ring with no substituent or one or more substituents on the ring, and the substituent is fluorine, alkyl or alkoxy;
Alk¹ is C₁₋₆ alkylidene;
Alk² is C₁₋₂ alkylidene;
Ar¹ is a benzene ring with no substituent or one or more substituents on the ring, and the substituent is fluorine, alkyl or alkoxy;
Ar² is a benzene ring or pyridine ring with no substituent or one or more substituents on the ring, and the substituent is fluorine, alkyl or alkoxy.

Preferably, the component (i) is a compound having the structure shown in Formula (II), or a pharmaceutically acceptable salt or isomer thereof:

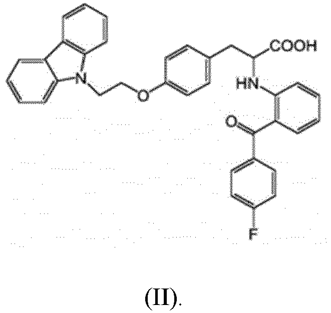

The pharmaceutically acceptable salt comprises an alkali metal salt, an alkaline earth metal salt, an ammonium salt and a salt of N⁺(C₁₋₄ alkyl)₄, the alkali metal salt comprises a potassium salt and a sodium salt, the sodium salt is chiglitazar sodium, and the alkaline earth metal salt comprises a calcium salt and a magnesium salt; and the isomer comprises S-configuration and R-configuration isomers.

The component (i) is a small molecule compound independently developed by Shenzhen Chipscreen Biosciences Co., Ltd. with completely independent intellectual property rights, and it has excellent hypoglycemic and hypolipidemic activity. The component (i) is disclosed and protected in Chinese patent No. CN1257893C, and this patent is introduced into the present disclosure in its entirety.

The component (ii), sodium-independent glucose transporter 2 (SGLT2), is a novel class of anti-diabetic drugs capable of inhibiting the reabsorption of glucose by the kidney, allowing excess glucose to be excreted from the urine and lowering blood glucose.

Preferably, the component (ii), sodium- dependent glucose transporter 2 (SGLT2) inhibitor, comprises Canagliflozin, Dapagliflozin, Empagliflozin, Ipragliflozin or Ipragliflozin L-Proline (a complex of Ipragliflozin and L-Proline), Luseogliflozin, Tofogliflozin, and Ertuglifolzin.

The component (i) and the component (ii) have a dosage in therapeutically effective amount, the dosage of component (i) is 1-100mg, preferably 12-48mg; and the dosage of component (ii) is 1-500mg, the therapeutically effective amount is selected depending on the specific type of sodium-dependent glucose transporter 2 (SGLT2) inhibitor, for example, the therapeutically effective amount of Canagliflozin is 50-300mg, the therapeutically effective amount of Dapagliflozin is 2.5-10mg, the therapeutically effective amount of Empaglifozin is 5-25mg, the therapeutically effective amount of Ipragliflozin L-Proline is 12.5-50mg, the therapeutically effective amount of Luseogliflozin is 1.25-5mg, the therapeutically effective amount of Tofogliflozin is 5-20mg, and the therapeutically effective amount of Ertuglifolzin is 2.5-15mg.

In another asepect, the present disclosure also provides a pharmaceutical composition comprising a component (i) and a component (ii), wherein,
the component (i) is a compound having the formula shown in general Formula (I), or a pharmaceutically acceptable salt or isomer thereof; the component (ii) is a sodium-dependent glucose transporter 2 (SGLT2) inhibitor: 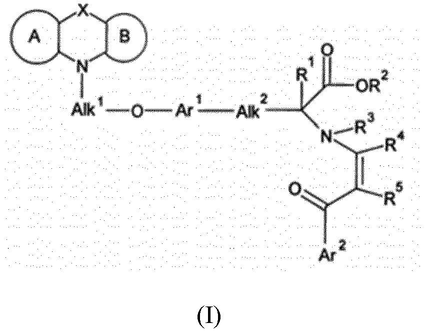
wherein rings A and B are respectively benzene rings, with no substituent or one or more substituents on the ring, and the substituent is fluorine, alkyl or alkoxy;
X is a covalent bond, O or S;
R¹ is H or alkyl;
R² is H or alkyl;
R³ is H or alkyl;
R⁴ and R⁵ are H or alkyl, respectively, or R⁴ and R⁵ together form a benzene ring with no substituent or one or more substituents on the ring, and the substituent is fluorine, alkyl or alkoxy;
Alk¹ is C₁₋₆ alkylidene;
Alk² is C₁₋₂ alkylidene;
Ar¹ is a benzene ring with no substituent or one or more substituents on the ring, and the substituent is fluorine, alkyl or alkoxy;
Ar² is a benzene ring or pyridine ring with no substituent or one or more substituents on the ring, and the substituent is fluorine, alkyl or alkoxy.

Preferably, the component (i) is a compound having the structure shown in Formula (II), or a pharmaceutically acceptable salt or isomer thereof;

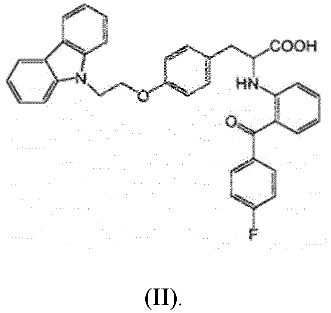

The pharmaceutically acceptable salt comprises an alkali metal salt, an alkaline earth metal salt, an ammonium salt and a salt of N⁺(C₁₋₄ alkyl)₄, the alkali metal salt comprises a potassium salt and a sodium salt, and the alkaline earth metal salt comprises a calcium salt and a magnesium salt; and the isomer comprises S-configuration and R-configuration isomers.

Preferably, the component (ii), sodium-dependent glucose transporter 2 (SGLT2) inhibitor, comprises Canagliflozin, Dapagliflozin, Empagliflozin, Ipragliflozin or Ipragliflozin L-Proline (a complex of Ipragliflozin and L-Proline), Luseogliflozin, Tofogliflozin, and Ertuglifolzin.

The component (i) and the component (ii) have a dosage in therapeutically effective amount, the dosage of component (i) is 1-100mg, preferably 12-48mg; and the dosage of component (ii) is 1-500mg, the therapeutically effective amount is selected depending on the specific type of sodium-dependent glucose transporter 2 (SGLT2) inhibitor, for example, the therapeutically effective amount of Canagliflozin is 50-300mg, the therapeutically effective amount of Dapagliflozin is 2.5-10mg, the therapeutically effective amount of Empaglifozin is 5-25mg, the therapeutically effective amount of Ipragliflozin L-Proline is 12.5-50mg, the therapeutically effective amount of Luseogliflozin is 1.25-5mg, the therapeutically effective amount of Tofogliflozin is 5-20mg, and the therapeutically effective amount of Ertuglifolzin is 2.5-15mg.

In a third aspect, the present disclosure provides a compound medicament comprising the pharmaceutical composition as described above, and one or more pharmaceutically acceptable excipients or carriers.

The compound medicament of the present disclosure can be prepared in a solid preparation or liquid preparation by a method generally used in the art, wherein the solid preparation includes tablets, capsules, granules, pills, powders and suppositories, and the like.

Suitable types of the pharmaceutically acceptable excipients or carriers include, but are not limited to: diluents, fillers, binders, disintegrants, lubricants, flow aids, pelletization additives, coating agents, wetting agents, solvents, co-solvents, suspension aids, emulsifiers, sweeteners, flavor modifiers, flavor masking agents, colorants, anti-caking agents, humectants, chelating agents, plasticizers, viscosity enhancers, antioxidants, preservatives, stabilizers, surfactants, and buffering agents, etc.

Examples of suitable pharmaceutically acceptable excipients or carriers include, but are not limited to: lactose, glucose, sucrose, sorbitol, mannitol, starch, gum arabic, calcium phosphate, alginate, tragacanth, gelatin, calcium silicate, microcrystalline cellulose, polyvinylpyrrolidone, cellulose, methyl cellulose, water, syrup, talc, magnesium stearate, mineral oil, methyl hydroxybenzoate and propyl hydroxybenzoate, and the like.

In a fourth aspect, the present disclosure provides a kit comprising the aforementioned pharmaceutical composition, wherein the component (i) and the component (ii) are unit preparations having the same or different specifications, respectively, and may be provided in separate containers.

In a fifth aspect, the present disclosure also provides a method of preventing and/or treating diabetes mellitus and/or a diabetic complication including cardiovascular disease, kidney disease, liver disease, comprising a step of administering a therapeutically effective amount of the aforementioned pharmaceutical composition, the compound medicament or the kit.

### Beneficial effects of the present disclosure:

In the present disclosure, leptin receptor gene-knockout diabetic mouse strain BKS-Leprem2Cd479/Nju (also known as db/db mice) was administrated with chiglitazar sodium in combination with SGLT2 inhibitor (Empagliflozin or Dapagliflozin) for two weeks, and the changes in glycemic and lipidemia index of the combination group in comparison with the monotherapy group or vehicle group were detected. The results showed that the combination of chiglitazar sodium with SGLT2 inhibitor was able to synergistically reduce blood glucose in db/db diabetic mice and also alleviate the weight gain induced by chiglitazar sodium, showing an unexpected synergistic effect. Also, the combination of chiglitazar sodium with SGLT2 inhibitor improved lipidemia index, particularly serum triglyceride levels.

Chiglitazar sodium is a PPARα/γ/δ receptor full agonist, which can sensitize insulin, reduce blood glucose and partially improve blood lipids; SGLT2 inhibitor can inhibit the reabsorption of glucose by the kidney, allow excess glucose to be excreted from the urine, reduce blood glucose and also effectively lose body weight. In terms of mode of action, at least three aspects would benefit from the combination of chiglitazar sodium and SGLT2 inhibitor, including: (1) SGLT2 inhibitor reduces the absorption of glucose in the body, and chiglitazar sodium increases insulin sensitivity, thereby reducing blood glucose from different pathways and achieving a synergistic effect; (2) chiglitazar sodium improves blood lipids and has the potential to treat related metabolic diseases, while the weight-losing effect of SGLT2 inhibitor also benefits the metabolism, and a synergistic effect of improving metabolic diseases is produced; (3) the weight-losing effect of SGLT2 inhibitor can offset the weight gain side effects of chiglitazar sodium to some extent, thereby benefiting diabetic patients with obesity complications.

Peroxisome proliferator-activated receptor (PPAR) agonists have the effects including alleviating insulin resistance, reducing the risk of new-onset diabetes, and protecting cardiovascular system, while sodium-glucose linked transporter-2 (SGLT2) inhibitors benefit cardiovascular disease and nephropathy. The component (i) used in the present disclosure is a PPAR full agonist drug, and clinical studies have shown its efficacy in reducing blood glucose and improving blood lipids in diabetic patients, as well as reducing systemic inflammation and having certain hepatic and renal protective effects, but having certain weight gain effect; while SGLT2 inhibitors have efficacy in reducing blood glucose, blood pressure, and body weight in diabetic patients, thereby bringing renal function and cardiovascular protection. Thus, it can be seen that the combination of the two classes of drugs can produce combined effects in controlling weight, lipids, blood pressure, hepatic and renal protection, in addition to more efficient in reducing blood glucose, thus bringing more benefits in the prevention and/or treatment of diabetes and its complications, including cardiovascular and cerebrovascular diseases, renal and liver diseases.

### Brief Description of the Drawings

Fig. 1 shows the effect on body weight and blood glucose of chiglitazar sodium in combination with empagliflozin, compared with monotherapy thereof.
Fig. 2 shows the effect on body weight and blood glucose of chiglitazar sodium in combination with dapagliflozin, compared with monotherapy thereof.
Fig. 3 shows the effect on blood lipids of chiglitazar sodium in combination with empagliflozin.
Fig. 4 shows the effect on blood lipids of chiglitazar sodium in combination with dapagliflozin.

### Specific Models for Carrying Out the Disclosure

The present disclosure discloses a drug combination and pharmaceutical composition thereof for the treatment of diabetes mellitus and its complications, which can be implemented by those skilled in the art by drawing on the contents disclosed herein with appropriate substitutions or modifications. In particular, it is noted that all similar substitutions and modifications will be apparent to those skilled in the art, and they are all considered to be included in the present disclosure. The applications described herein have been described by way of preferred examples, and it is apparent that interested persons are able to implement and apply the techniques of the present disclosure by making changes or appropriate alterations and combinations to the applications described herein without departing from the content, spirit and scope of the present disclosure.

The present disclosure is further described below by means of non-limiting examples which are not intended to limit the scope covered by the present disclosure.

Unless otherwise defined, all technical and scientific terms used herein have the same meaning as commonly understood by those of ordinary skilled in the art.

### Example 1: Comparison of effects on body weight and blood glucose between chiglitazar sodium in combination with empagliflozin and monotherapy thereof

Experimental design: the effects on body weight and blood glucose of single and combined medication were investigated, by oral administration of chiglitazar sodium, empagliflozin, or a combination thereof to mice.

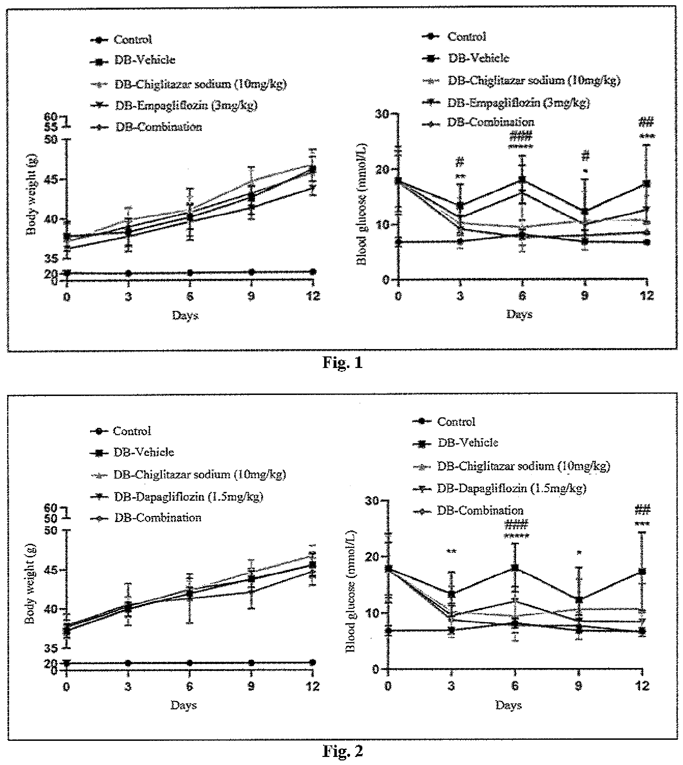

Experimental procedure: 6-Week-old db/db male mice were orally administered once a day with 100 µL of vehicle (0.1% sodium carboxymethylcellulose), 10 mg/kg chiglitazar sodium, 3 mg/kg empagliflozin, or a combination of chiglitazar sodium and empagliflozin at the same doses simultaneously, respectively, according to body weight. Wild-type C57BLKS/Jnju mice were used as normal control (administered with the vehicle). The period of administration was 14 days, and the animals were treated and dissected on Day 15. Starting from the administration, the mice were subjected to weighing and measurement of fasting blood glucose (4-6 hours of fasting) every 3 days. The results of the experiment were shown in Fig. 1.

The results showed that chiglitazar sodium and empagliflozin alone could reduce blood glucose to a certain extent, and the combination of the two generated an unexpected synergistic effect, it could alleviate the weight gain caused by chiglitazar sodium on the one hand, and it had better blood glucose lowering effect on the other, even to a level close to that of the normal control group.

### Example 2: Comparison of effects on body weight and blood glucose between chiglitazar sodium in combination with dapagliflozin and monotherapy thereof

Experimental design: the effects on body weight and blood glucose of single and combined medication were investigated, by oral administration of chiglitazar sodium, dapagliflozin, or a combination thereof to mice.

Experimental procedure: 6-Week-old db/db male mice were orally administered once a day with 100 µL of vehicle (0.1% sodium carboxymethylcellulose), 10 mg/kg chiglitazar sodium, 1.5 mg/kg dapagliflozin, or a combination of chiglitazar sodium and dapagliflozin at the same doses simultaneously, respectively, according to body weight. Wild-type C57BLKS/Jnju mice were used as normal control (administered with vehicle). The period of administration was 14 days, and the animals were treated and dissected on Day 15. Starting from the administration, the mice were subjected to weighing and measurement of fasting blood glucose (4-6 hours of fasting) every 3 days. The results of the experiment were shown in Fig. 2.

The results showed that chiglitazar sodium and dapagliflozin alone could reduce blood glucose to a certain extent, and the combination of the two generated an unexpected synergistic effect, it could alleviate the weight gain caused by chiglitazar sodium on the one hand, and it had better blood glucose lowering effect on the other, even to a level close to that of the normal control group.

### Example 3: Effect on blood lipids of chiglitazar sodium in combination with empagliflozin

Experimental design: the effects on total cholesterol (TC) and triglyceride (TG) in mouse serum of single and combined medication were investigated, by oral administration of chiglitazar sodium, empagliflozin, or a combination thereof to diabetic mice.

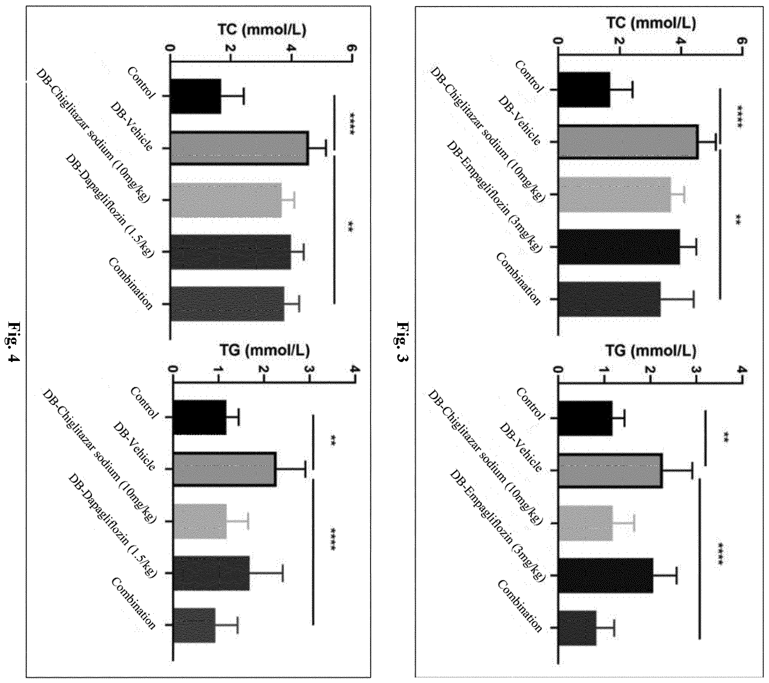

Experimental procedure: 6-Week-old db/db male mice were orally administered once a day with 100 µL of vehicle (0.1% sodium carboxymethylcellulose), 10 mg/kg chiglitazar sodium, 3 mg/kg empagliflozin, or a combination of chiglitazar sodium and empagliflozin at the same doses simultaneously, respectively, according to body weight. Wild-type C57BLKS/Jnju mice were used as normal control (administered with vehicle). The period of administration was 14 days, and the animals were treated and dissected on Day 15. Whole blood was collected, serum was centrifuged and serum TC and TG were measured by biochemical analyzer. The experimental results were shown in Fig. 3.

The results showed that chiglitazar sodium and empagliflozin alone could reduce the serum total cholesterol and triglyceride levels to a certain extent, wherein chiglitazar sodium showed relatively better effect; while the combination of the two showed better effect in reducing the serum total cholesterol and triglyceride levels, a synergistic effect was generated.

### Example 4: Effect on blood lipids of chiglitazar sodium in combination with dapagliflozin

Experimental design: the effects on total cholesterol (TC) and triglyceride (TG) in mouse serum of single and combined medication were investigated, by oral administration of chiglitazar sodium, dapagliflozin, or a combination thereof to diabetic mice.

Experimental procedure: 6-Week-old db/db male mice were orally administered once a day with 100 µL of vehicle (0.1% sodium carboxymethylcellulose), 10 mg/kg chiglitazar sodium, 1.5 mg/kg dapagliflozin, or a combination of chiglitazar sodium and dapagliflozin at the same doses simultaneously, respectively, according to body weight. Wild-type C57BLKS/Jnju mice were used as normal control (administered with vehicle). The period of administration was 14 days, and the animals were treated and dissected on Day 15. Whole blood was collected, serum was centrifuged and serum TC and TG were measured by biochemical analyzer. The experimental results were shown in Fig. 4.

The results showed that chiglitazar sodium and dapagliflozin alone could reduce the serum total cholesterol and triglyceride levels to a certain extent, wherein chiglitazar sodium showed relatively better effect; while the combination of the two showed better effect in reducing the serum total cholesterol and triglyceride levels, a synergistic effect was generated.

In the present disclosure, it was verified by Examples 1 to 4 that the combination of chiglitazar sodium with SGLT2 inhibitor, empagliflozin or dapagliflozin, could synergistically reduce blood glucose in db/db diabetic mice and also alleviate the weight gain induced by chiglitazar sodium, showing an unexpected synergistic effect. Also, the combination of chiglitazar sodium with SGLT2 inhibitor improved lipidemia index, particularly serum triglyceride levels. Chiglitazar sodium is a peroxisome proliferator-activated receptor (PPAR) agonist, and peroxisome proliferator-activated receptor (PPAR) agonists have the effects including alleviating insulin resistance, reducing the risk of new-onset diabetes, and protecting cardiovascular system, while sodium-glucose linked transporter-2 (SGLT2) inhibitors benefit cardiovascular disease and nephropathy. Clinical studies show that chiglitazar sodium has efficacy in reducing blood glucose and improving lipids in diabetic patients, as well as reducing systemic inflammation and having certain hepatic and renal protective effects, but having certain weight gain effect; while SGLT2 inhibitors have efficacy in reducing blood glucose, blood pressure, and body weight in diabetic patients, thereby bringing renal function and cardiovascular protection. Thus, it can be seen that the combination of the two classes of drugs can produce combined effects in controlling weight, lipids, blood pressure, hepatic and renal protection, in addition to more efficient in reducing of blood glucose, thus bringing more benefits to the prevention and/or treatment of diabetes and its complications, including cardiovascular and cerebrovascular diseases, renal and liver diseases.

The present disclosure has been described in detail above; specifically, the principles and implementation of the present disclosure are illustrated with preferred examples. The description of the above examples is only intended to assist in understanding the method of the present disclosure and its core ideas, including the best mode, and also to enable any person skilled in the art to practice the present disclosure, including manufacture and use of the disclosure, and method to implement any combinations. It should be noted that for a person of ordinary skilled in the art, there are several improvements and modifications that can be made to the present disclosure without departing from the principles of the present disclosure, and these improvements and modifications also fall within the scope of protection of the claims of the present disclosure. The scope of the patent protection of the present disclosure is limited by the claims and may include other embodiments that can be thought of by a person skilled in the art. If these other embodiments have structural elements that are not different from the literal representation of the claims, or if they include equivalent structural elements that are not substantially different from the literal representation of the claims, then these other embodiments shall also be included in the scope of the claims.

## Claims

1. Use of a component (i) in combination with a component (ii) in the manufacture of a medicament for the prevention and/or treatment of diabetes mellitus and/or a diabetic complication, wherein,
the component (i) is a compound having the formula shown in general Formula (I), or a pharmaceutically acceptable salt or isomer thereof; the component (ii) is a sodium-dependent glucose transporter 2 (SGLT2) inhibitor: 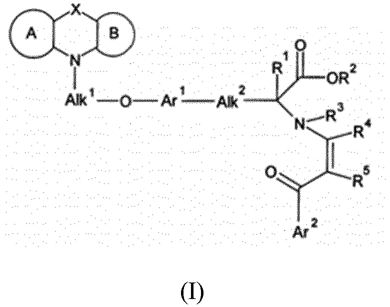
wherein ring A and ring B are respectively benzene rings, with no substituent or one or more substituents on the ring, and the substituent is fluorine, alkyl or alkoxy;
X is a covalent bond, O or S;
R¹ is H or alkyl;
R² is H or alkyl;
R³ is H or alkyl;
R⁴ and R⁵ are H or alkyl, respectively, or R⁴ and R⁵ together form a benzene ring with no substituent or one or more substituents on the ring, and the substituent is fluorine, alkyl or alkoxy;
Alk¹ is C₁₋₆ alkylidene;
Alk² is C₁₋₂ alkylidene;
Ar¹ is a benzene ring with no substituent or one or more substituents on the ring, and the substituent is fluorine, alkyl or alkoxy;
Ar² is a benzene ring or pyridine ring with no substituent or one or more substituents on the ring, and the substituent is fluorine, alkyl or alkoxy.

2. The use according to claim 1, wherein the component (i) is a compound having the structure shown in Formula (II), or a pharmaceutically acceptable salt or isomer thereof:

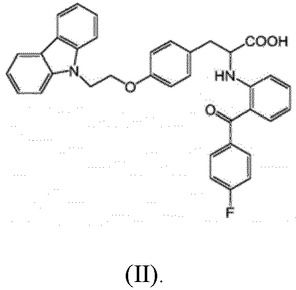

3. The use according to claim 1 or 2, wherein the pharmaceutically acceptable salt comprises an alkali metal salt, an alkaline earth metal salt, an ammonium salt and a salt of N⁺(C₁₋₄ alkyl)₄, the alkali metal salt comprises a potassium salt and a sodium salt, and the alkaline earth metal salt comprises a calcium salt and a magnesium salt; and the isomer comprises S-configuration and R-configuration isomers.

4. The use according to any one of claims 1 to 3, wherein the component (ii), sodium-dependent glucose transporter 2 (SGLT2) inhibitor, comprises Canagliflozin, Dapagliflozin, Empagliflozin, Ipragliflozin or Ipragliflozin L-Proline (a complex of Ipragliflozin and L-Proline), Luseogliflozin, Tofogliflozin, and Ertuglifolzin.

5. The use according to any one of claims 1 to 4, wherein the component (i) and the component (ii) have a dosage in therapeutically effective amount, the dosage of component (i) is 1-100mg, and the dosage of component (ii) is 1-500mg.

6. The use according to any one of claims 1 to 5, wherein the diabetes mellitus is type II diabetes mellitus, and the diabetic complication comprises cardiovascular disease, kidney disease and liver disease.

7. A pharmaceutical composition, **characterized in that**, the pharmaceutical composition comprises a component (i) and a component (ii), wherein,
the component (i) is a compound having the formula shown in general Formula (I), or a pharmaceutically acceptable salt or isomer thereof; the component (ii) is a sodium-dependent glucose transporter 2 (SGLT2) inhibitor: 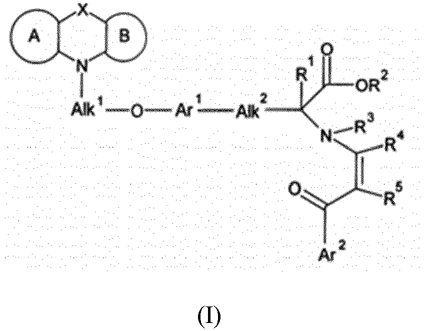
wherein rings A and B are respectively benzene rings, with no substituent or one or more substituents on the ring, and the substituent is fluorine, alkyl or alkoxy;
X is a covalent bond, O or S;
R¹ is H or alkyl;
R² is H or alkyl;
R³ is H or alkyl;
R⁴ and R⁵ are H or alkyl, respectively, or R⁴ and R⁵ together form a benzene ring with no substituent or one or more substituents on the ring, and the substituent is fluorine, alkyl or alkoxy;
Alk¹ is C₁₋₆ alkylidene;
Alk² is C₁₋₂ alkylidene;
Ar¹ is a benzene ring with no substituent or one or more substituents on the ring, and the substituent is fluorine, alkyl or alkoxy;
Ar² is a benzene ring or pyridine ring with no substituent or one or more substituents on the ring, and the substituent is fluorine, alkyl or alkoxy.

8. The pharmaceutical composition according to claim 7, wherein the component (i) is a compound having the structure shown in Formula (II), or a pharmaceutically acceptable salt or isomer thereof;

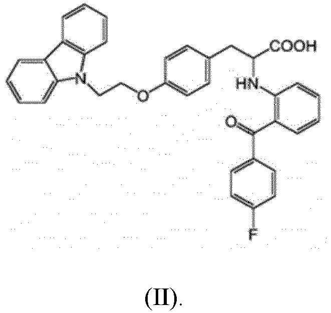

9. The pharmaceutical composition according to claim 7 or 8, wherein the pharmaceutically acceptable salt comprises an alkali metal salt, an alkaline earth metal salt, an ammonium salt and a salt of N⁺(C₁₋₄ alkyl)₄, the alkali metal salt comprises a potassium salt and a sodium salt, and the alkaline earth metal salt comprises a calcium salt and a magnesium salt; and the isomer comprises S-configuration and R-configuration isomers.

10. The pharmaceutical composition according to any one of claims 7 to 9, wherein the component (ii), sodium-dependent glucose transporter 2 (SGLT2) inhibitor, comprises Canagliflozin, Dapagliflozin, Empagliflozin, Ipragliflozin or Ipragliflozin L-Proline (a complex of Ipragliflozin and L-Proline), Luseogliflozin, Tofogliflozin, and Ertuglifolzin.

11. The pharmaceutical composition according to any one of claims 7 to 10, wherein the component (i) and the component (ii) have a dosage in therapeutically effective amount, the dosage of component (i) is 1-100mg, and the dosage of component (ii) is 1-500mg.

12. A compound medicament, **characterized in that**, the compound medicament comprises the pharmaceutical composition according to any one of claims 7 to 11, and one or more pharmaceutically acceptable excipients or carriers.

13. The compound medicament according to claim 11, which is in a dosage form including tablets, capsules, granules, pills, powders and suppositories.

14. A kit, **characterized in that**, the kit comprises the pharmaceutical composition according to any one of claims 7 to 11.

15. The kit according to claim 14, **characterized in that**, the component (i) and the component (ii) are unit preparations having the same or different specifications, respectively, and may be provided in separate containers.
